# EUROPEAN PATENT SPECIFICATION

(11) **EP 1 852 057 B1**
(45) Date of publication and mention of the grant of the patent: **12.05.2010**
(21) Application number: 07015886.0
(22) Date of filing: 20.03.2003
(51) Int. Cl.: A61B 3/12

(54) **System and method for visualizing fluid flow through vessels**
System und Verfahren zur Ansicht des Flüssigkeitsstroms durch Gefäße
Système et procédé pour visualiser l'écoulement de fluides dans les vaisseaux

(30) Priority: 20.03.2002 US 365547 P
(43) Date of publication of application: 07.11.2007
(62) Divisional of application: 03744293.6
(73) Proprietor: Novadaq Technologies Inc., Mississauga, ON L4W 4L5 (CA)
(72) Inventor: Flower, Robert, Hunt Valley, MD 21030 (US); Kling, Ronn, Warrenton, VA 20187 (US)
(74) Representative: Roberts, Gwilym Vaughan

(56) References cited:
- WO-A-01/28405
- US-A- 5 394 199
- US-A- 6 158 864
- US-B1- 6 350 238

## Description

This invention relates to a computer aided system and method for visualizing flow in fluid carrying vessels in animal tissue and more particularly to a system and method for visualizing and identifying a direction of blood-flow in vessels supplying blood to a lesion, such as a neovascular lesion associated with age-related macular degeneration, to assist diagnosis and treatment of such lesions.

US 6,350,238 discloses a method for real-time display of ultrasound in slow motion.

### BACKGROUND OF THE INVENTION

Lesions are normally defined as an abnormal tissue structure located in an organ or other body part, and are often a manifestation of a harmful condition, disease or illness. Lesions may take many specific forms, such as choroidal neovascularizations ("CNVs") which are found in the eye. In general, any abnormal vasculature in a body is a type of lesion.

A CNV is one manifestation of age related macular degeneration ("AMD") and is a common cause of vision loss in people over the age of 50. In 1995, of the estimated 34 million people in the United States who were age 65 or older, approximately 1.7 million had some visual impairment resulting from AMD. Timely diagnosis and treatment of CNV is an important therapeutic objective because permanent vision loss can result if hemorrhage of the CNV occur.

### Visualization of the CNV

Visualization is a process of obtaining and viewing an angiographic image representation of the blood vessels in a region of interest following introduction of a visual enhancement material into such vessels.

Ordinarily, the choroidal vasculature of the eye cannot be readily visualized because the pigments in the retinal pigment epithelium ("RPE") layer (sandwiched between the sensory retina and the choriocapillaris) and the pigments in the choroid do not readily transmit visible light wavelengths. Therefore, even sodium fluorescein angiography, routinely used to demonstrate the retinal vascular blood flow and whose excitation and emission spectra are comprised of visible wavelengths, shows only a faint diffuse flush resulting from vascular staining with fluorescein dye as it transits through the choroidal circulation and before dye enters the more superficial retinal vasculature.

Another methodology, termed indocyanine green ("ICG") dye angiography ("ICGA"), is used for routinely visualizing choroidal blood flow. It is based on use of the near-infrared fluorescent light wavelengths emitted by ICG, which readily penetrate the pigmented ocular tissues. The impetus for developing ICGA about 30 years ago was to provide a tool for studying choroidal hemodynamics and blood flow physiology, but during the past 5 years, as a result of the equipment to implement it having became commercially available, its clinical use has become wide spread for detecting and monitoring AMD-associated CNV.

Visualizing and monitoring CNV blood flow has become an important part of diagnosing and treating CNV, especially when the lesions are directly beneath or immediately adjacent to the foveal area of the retina.

The commercial availability of the scanning laser ophthalmoscope (SLO) contributed to an increasing interest in ICGA. Compared to the predominantly available commercial ICGA systems based on fundus camera optics-capable of acquiring images at a rate of about one per second-the SLO afforded the ability to perform high-speed imaging. Ready access to high-speed ICG image acquisition systems was an important component of interest in a new AMD treatment modality, namely CNV feeder vessel photocoagulation treatment.

### CNV Feeder Vessel Photocoagulation

A recent approach being pursued is so-called feeder vessel ("FV") photocoagulation, wherein the afferent vessel supplying blood to the sub-foveal CNV is photocoagulated at a site outside the foveal area. So far, FV photocoagulation has proven to be an apparently effective treatment for CNVs, often resulting in improved visual acuity - a result not usually associated with other forms of treatment that have been used or considered to date. This treatment method is of particular interest for occult subfoveal CNV, for which there is no other treatment approach at this time.

As mentioned earlier, FVs are those vessels identified as supplying blood to an area of CNV. The length of these vessels are on the order of one-half to several millimeters long, and are thought to lie in Sattler's layer of the choroid (See: Flower R. W., ExperimentalStudies of Inclocyanine Greert. Dye-Erthczh. ced Plzotocoagulation of Choroidal Neovascularizatio 71 Feeder Vessels, Am. J. Ophthalmol., 2000,129 : 501-512). Note that Sattler's layer is the middle layer of choroidal vessels containing the arterial and venous vessels that feed and drain blood from the thin layer of choroidal capillary vessels lying just beneath the sensory retina. Following visualization of a CNV feeder vessel, e.g. by ICGA, photocoagulation of the feeder vessel is then performed in order to reduce or stop the flow of blood to the CNV.

Studies indicate often-dramatic resolution of the CNV-associated retinal edema and stabilization or even improvement in visual acuity, often within hours of feeder vessel photocoagulation.

A major drawback to FV treatment as currently practiced is that it is necessarily limited by the accurate identification and visualization of the FVs that supply blood to a given lesion. Thus, the success of both FV photocoagulation, and alternatives such as FV dye-enhanced photocoagulation ("DEP"), hinges on proper identification of the FVs. Conventionally, a single angiogram following single large ICG bolus injection are used to locate and identify feeder vessels. This single angiogram (one sequence of angiographic images) using conventional methods is insufficient to obtain proper blood flow data, thus necessitating the use of multiple angiographic sequences on the same eye. Furthermore, choroidal tissue staining produced by a single large dye bolus using conventional methods produces images of such poor contrast that accurate FV identification is difficult. Thus, after performing one angiogram, it is often necessary to perform a second angiogram in order to obtain a second set of angiographic images. This is often necessary because the first set of images is of insufficient quality to identify a FV. Thus, the parameters are adjusted for the second angiogram, based on the outcome of the first set of images, in order to acquire better quality images and thus adequate data about lesion blood flow. Accordingly, if the second set of angiographic images from the second angiogram is still inadequate for FV identification, a third angiogram must be performed. In addition, repeated angiogram studies result in declining contrast and image quality.

Even after obtaining technically adequate angiographic image sequences, sure identification of CNV FVs (as opposed to the draining vessels) is often difficult, especially when dye transits the FVs in pulsatile fashion and in the presence of significant background dye fluorescence from underlying choroidal vasculature. Under such circumstances, it is usually not possible to identify either specific feeder vessels or the direction of flow through them.

Current techniques for identifying and visualizing FVs are limited. A need therefore exists for, and it is an object of the present invention to offer, improved identification of feeder vessels and visualization of the direction of blood flow through vessels.

### SUMMARY OF THE INVENTION

It is an objective of this invention to provide a system and method for visualizing fluid carrying vessels in animal tissue and more particularly to provide a system and method for visualizing and identifying a direction of blood-flow in vessels supplying blood to a lesion

Another objective of this invention is to significantly increase the frequency with which potentially treatable feeder vessels of a lesion can be detected. It is a further object of the invention to significantly increase the frequency with which potentially treatable feeder vessels of juxta- and sub-foveal age-related macular degeneration associated CNV can be detected.

The method of the invention is based on the premise that one can more readily identify FVs, and the visualization of dye through blood vessels is generally augmented by, serially displaying a series of angiographic images, of the dye flowing through the area of interest, repetitively, while being able to manipulate certain parameters in real-time. The serial display of images is generally referred to as phi-motion.

In accordance with one aspect of the present invention, there is provided a method as set out in claim 1 for visualizing fluid flow through vessels having a visualizing composition flowing therethrough. The method includes the following steps. Selecting from a sequence of angiographic images a subsequence of angiographic images. Reading a plurality of dynamic parameters, said dynamic parameters for controlling display of said angiographic images. Serially displaying said subsequence repetitively in accordance with said dynamic parameters. And providing an interface for dynamic user update of said dynamic parameters while displaying said subsequence. This method is referred to as Interactive Phi-Motion ("IPM").

In one embodiment of the invention, dynamic parameters consisting of speed, interval, direction and pixel brightness are used and are dynamically adjustable. Preferably, the pixel brightness relationship, between collected and displayed intensity values, is represented by and manipulable through a Look-Up Table ("LUT").

Also provided is an apparatus for carrying out the inventive method, a computer program product comprising a memory with code embodied thereon corresponding to the method, and a computer readable memory storing instructions corresponding to the method.

According to an aspect, which does not constitute a portion of the invention, a method is provided for treating a lesion with at least one blood vessel feeding blood to it. This method comprises: administering a visualizing composition comprising a fluorescent dye; capturing a plurality of angiographic images of a pre-selected area surrounding the lesion; visualizing the flow of said visualizing composition through said lesion using IPM; identifying said blood vessel; applying energy to said blood vessel, of a type and an amount sufficient to reduce the rate of blood flow through said blood vessel.

Advantageously, the methods of the present invention offer many benefits over conventional methods. First, by displaying the angiographic images in phi-motion and manipulating the dynamic parameters in real-time, visualization of the vessels of interest and the dye front entering and filling up the vessels can be optimized. Second, only one set of high-speed angiographic images need be taken, thus obviating the need to adjust parameters between captures in order to optimize visualization based on the previous angiogram. Third, by the ability to visualize the dye wavefront, the direction of flow in the vessel can be determined with certainty, thereby assuring that afferent and not efferent vessels are treated.

Further aspects of the invention are defined in the accompanying independent claims.

Still further advantages and benefits of the present invention will become apparent to those of ordinary skill in the art upon reading and understanding the following detailed description of the preferred embodiments.

### BRIEF DESCRIPTION OF THE DRAWINGS

These and other features of the preferred embodiments of the invention will become more apparent in the following detailed description in which reference is made to the appended drawings wherein:

Figure 1 illustrates, in flow chart form, a method of treatment of a blood vessel supplied lesion by photocoagulation incorporating the use of IPM.

Figure 2 illustrates, in flow chart form, the method of IPM.

Figure 3 illustrates the default LUT according to one embodiment ofIPM.

Figure 4 illustrates a modified LUT according to one embodiment of IPM.

Figure 5 illustrates a second modified LUT according to one embodiment of IPM.

Figure 6 illustrates a system overview diagram of a preferred embodiment of the diagnoses and treatment of a patient with an AMD-related CNV using IPM.

Figure 7 illustrates a graphical user interface of a preferred embodiment of IPM.

### DESCRIPTION OF THE PREFERRED EMBODIMENTS

In the following description, numerous specific details are set forth to provide a thorough understanding of the invention. However, it is understood that the invention may be practiced without these specific details. In other instances, well-known processes have not been described or shown in detail in order not to obscure the invention. In the description and drawings, like numerals refer to like structures or and/or processes.

The methods of the present invention are claimed and described herein as a series of steps. It should be understood that these methods and associated steps may be performed in any logical order. Moreover, the methods may be performed alone, or in conjunction with other procedures and treatments administered before or after such methods and steps set forth herein without departing from the scope of the invention. Further, it is contemplated that the term animals as used herein includes, but is not limited to, humans.

Lesions, such as AMD-related CNV, have been shown to be successfully treated by photocoagulation or DEP of the blood vessel that feeds the lesion. Successful treatment, however, is predicated on the accurate and precise identification of the FV that supplies blood to the lesion. To this end, it is advantageous to both identify vessels that lead to the lesion and the direction of blood through that vessel, in order to determine whether it is a feeding (afferent) or draining (efferent) vessel. The possibility of achieving even more effective treatment of lesions, such as AMD-related CNV, lies in the improved visualization and identification of FVs using Interactive Phi-Motion prior to treatment. IPM provides improved visualization of dye flow through blood vessels, in a region of interest, by playing a subsequence of previously captured high-speed angiographic images repetitively, such as in a continuous loop, while providing the ability to dynamically manipulate certain parameters.
By dynamically changing these parameters, one can find the precise combination of such parameters that will optimize the visualization of FVs for each given case. This is important as the degree to which these parameters need to be adjusted will vary with each set of angiograms, since visualization will vary depending on many variables, such as the specific patient, dose of the visualizing dye, speed at which the images were captured... etc.

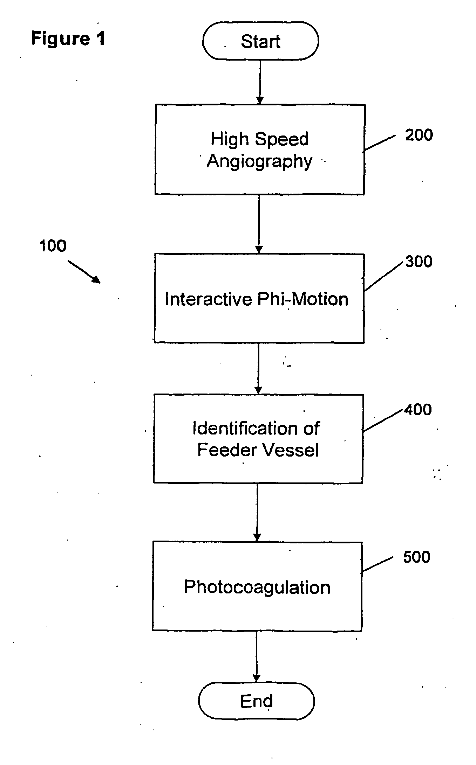

Referring to figure 1, one aspect, which does not constitute a portion of the present invention, provides a method for treating a lesion in an animal 100. For the method to be effective, the lesion should further have a blood vessel that carries blood into the lesion. The inventive method includes, but is not limited to, the following steps. Performing high-speed angiography on the lesion 200. Executing Interactive Phi-Motion 300. Identifying the FV 400. Photocoagulating the FV 500. In a preferred embodiment of the invention, the method 100 is used to treat AMD related CNVs in humans.

High-speed angiography 200 is performed using any suitable visualizing composition and obtaining high-speed images showing the visualizing composition filling the vessels in the region of interest. Preferably, a CNV and its associated feeder vessels are visualized using Indocyanine Green Dye Fluorescence Angiography. ICG dye is administered to the subject intravenously and allowed to perfuse through the subject's vasculature. Visualization is preferably effected by irradiating the area of interest with a laser light of a type and in an amount sufficient to cause the ICG dye to fluoresce.

A preferred dosage of ICG, for visualizing FVs, is about 7.5 mg administered at a concentration of about 25nig/ml in a volume of approximately 0.3ml administered intravenously. Only one bolus is required per imaging sequence but multiple boluses may be employed. A concentration of about 0.025mg/ml in blood theoretically produces the most fluorescence from the fundus of a mammalian eye. Additionally, in some embodiments of the invention, administration of ICG intravenously is followed by a 5ml saline flush. The saline flush is used to rapidly push the bolus out of the cubital vein and into the vasculature within the thoracic cavity.

Activation of the dye is preferably effected using a laser light source in the range of about 780nm-830nm. When visualizing a CNV and its associated feeder vessels in a mammalian eye, the laser light used to excite the dye preferably irradiates a target site of about 1cm² using about 20-100 mW of average power, although up to 230mW can be used. Irradiation of the target area with laser light is preferably effected for about 10-20 seconds.

Capturing the sequence of high-speed images of the fluorescing vasculature can be accomplished by numerous means which are known and will be apparent to a person skilled in the art. Images are preferably captured as high-speed angiographic images on a CCD camera and stored in memory as one subject sequence. Using the preferred dosages above, high-speed images are typically recorded at a preferred rate of about 30 frames/sec for 10-20secs in order to capture the fluorescence filling the vessel. According to this preferred embodiment of the invention, a typical subject sequence therefore is comprised of about 300 images.

While the above represents the preferred parameters for capturing high-speed angiographic images of a CNV, it is well known in the art that other dosages, light parameters and capture speeds are also effective to produce fluorescence in the eye such that the CNV and its associated feeder vessels can be visualized and angiographically captured.

Following the capture of high-speed angiographic images of the lesion and its associated FVs 200, Interactive Phi-Motion 300 is then performed on the sequence of high-speed images. Phi-motion is a phenomenon first identified by Wertheimer in 1912, it refers to the visual perception of motion where none exists, like a cinematic. By utilizing phi-motion, in conjunction with dynamically manipulable parameters, IPM 300 allows for the subsequent improved identification and visualization of an FVs and most notably the direction of blood flow through the FV 400.

Following identification of an FV suitable for treatment, photocoagulation of the vessel feeding the lesion is performed 500. Targeting of the photocoagulation treatment beam is based upon the information previously derived from visualization and identification of the lesion and its associated FVs 400 through IPM 300. Photocoagulation is effected by applying radiation of a kind and amount sufficient to effect an occlusion of the target vessel. It is believed that such occlusion occurs by increasing the temperature of the feeder vessel, resulting in either cauterization of the vessel or clotting of the blood within the vessel. As a result, the rate of blood flow through the vessel is reduced.

In a preferred embodiment of the invention, DEP is performed by previously injecting the subject with a radiation absorbing dye such as ICG dye. Photocoagulation is thus enhanced by utilizing the radiation absorbing properties of the ICG dye to perform dye enhanced photocoagulation of the FV. Preferably, an approximately 810nm treatment laser is used at about 400-600mW for about 1.0-1.5 seconds. This produces about 0.4J-0.9J of energy sufficient to photocoagulate the vessel in the presence of ICG.

The lesion is therefore attacked by cutting off the lesion's blood supply. This has the effect of starving the lesion and immediately reducing the hemodynamic pressure. Typical parameters for photocoagulation and DEP have been provided as preferred values and should not be construed as a limitation on the claims of the present invention

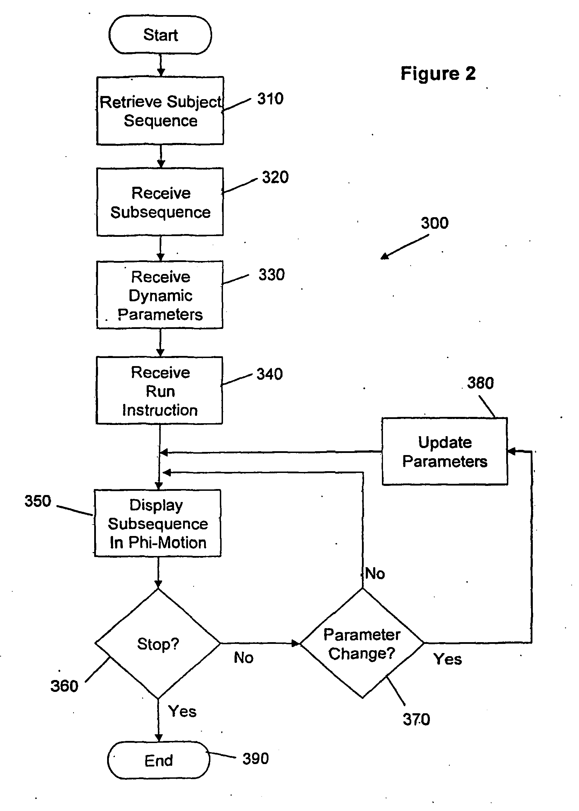

Referring to figure 2, another aspect of the present invention provides a method for visualizing blood flow through blood vessels using IPM 300. IPM is comprised of the following steps. A subject sequence of previously captured high-speed angiographic images is retrieved 310. Limits defining a sequential subsequence, representing the period of interest, are received 320. Dynamic parameters are then received 330. Once a run instruction is received 340, the subsequence is displayed in phi-motion 350 until stopped 360. During this display 350, parameter changes 370 are dynamically updated 380 in response to changes, by a user, in said dynamic parameters.

The subject sequence is typically retrieved from memory in response to a user selecting a specific sequence identifier. The subject sequence of previously captured high-speed angiographic images which is retrieved 310 for IPM should contain the period of interest. Therefore, the sequence should show the dye front entering and filling up the vessels in the lesion. Typically, a subject sequence is selected by a user and retrieved from a memory means.

Limits representing a subsequence of the subject sequence is then received 320. Typically, these limits are selected by a user by defining a frame and a number of frames surrounding, following or preceding the defined frame, or preferably, by definirig a first and last frame. The subsequence should define the period of interest, namely, the subsequence should contain that portion of the sequence that shows the visualizing dye entering the vessels, particularly the FV, associated with the lesion. The subsequence will be the only portion of the sequence undergoing IPM, thus, the longer the subsequence, the longer the IPM will take. Typically, using the preferred capture speeds and parameters above, about 30 frames are defined as the subsequence although any number of frames may be chosen depending on what the user wishes to visualize.

Following the defining of the subsequence, the dynamic parameters are received 330. In a preferred embodiment of the invention, there are four dynamic parameters associated with IPM: speed, interval, direction and pixel brightness. The dynamic parameters can be updated in real time in response to user input at any time, including during the display in phi-motion.

The speed parameter defines the speed at which the angiographic images are displayed in phi-motion. Typically, the speed parameter is quantified in frames per second, one frame representing one angiographic image in the subsequence.

The interval parameter defines whether every image in the subsequence will be displayed. For example, in one embodiment of the invention, entering a value of two (2) will result in the display of every second image in the subsequence in phi-motion. The interval parameter may thus be adjusted to effect a display of every n^{th} image (i.e. second, third, fourth...etc.) during phi-motion display.

The direction parameter defines the direction of the phi-motion display. In a preferred embodiment of the invention, the direction parameter can define running phi-motion in a continuous forward loop, continuous backward loop or continuous bounce. Selecting continuous bounce would result in a continuous display, in phi-motion, of the subsequence of angiographic images from first image to last image, last to first, first to last...etc.

The brightness parameter defines the relationship between collected intensity values and displayed intensity values. By manipulating the brightness parameter, pixels having a specific intensity on the collected angiographic image are adjusted to a different intensity on the phi-motion displayed angiographic image.

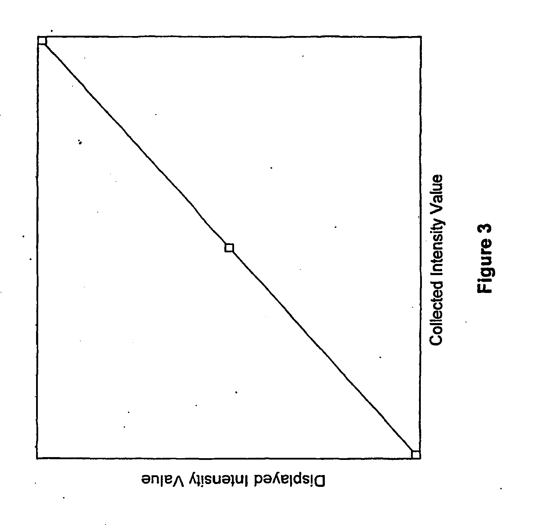

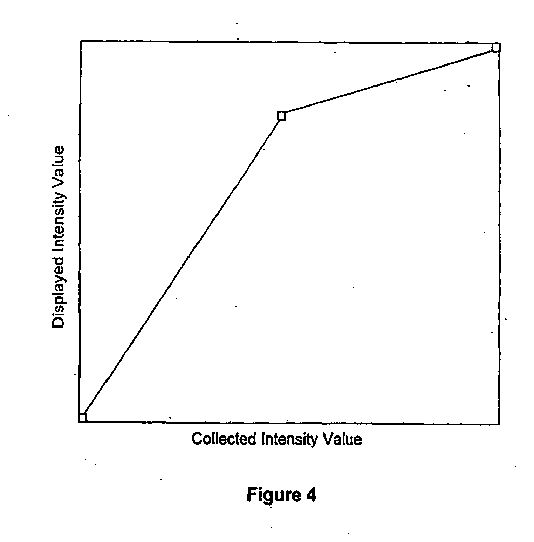

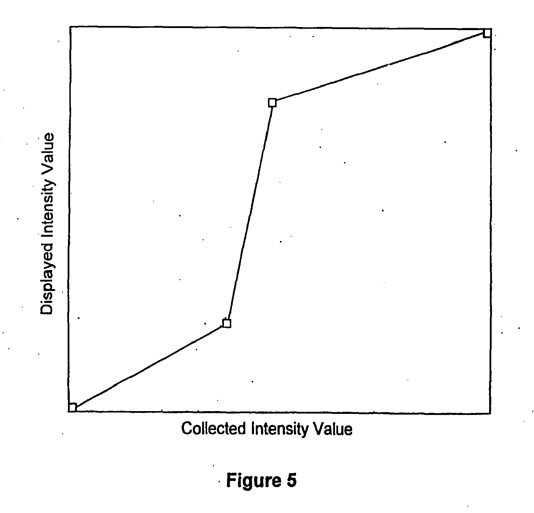

Referring to figure 3, in a preferred embodiment of the invention, the brightness parameter is received and adjusted by manipulation of a Look-Up Table. The x-axis represents the collected intensity value and the y-axis represents the displayed intensity value. Typically, by default, the brightness parameter is set so that the collected and displayed intensity values are directly proportional and represented by a linear line with a slope of one. In this example, there are three control points in the LUT. There are two control points at the opposite corners and one control point in the middle. By displacing the control points, the user can manipulate the relationship between collected and displayed intensity values. Referring to figure 4, the control point in the middle of line has been moved upwards in response to user manipulation, typically by clicking and dragging a pointing device. The new line, still fixed at opposite corners is now comprised of 2 line segments and defines a new and adjusted relationship between the collected and displayed intensity values. Referring to figure 5, new points may also be added by a user, such as by clicking a pointing device on part of the LUT, in order to further segment the line and further manipulate the relationship between collected and displayed intensity values. It is important to note that in this embodiment of the invention, it is not possible to move successive points on the line behind another, that is, to make the line double back in such a way that one collected intensity value corresponds to more than one displayed intensity value.

These parameters can be dynamically modified during phi-motion display in order to better visualize the blood flow through the region of interest. This allows for improved identification of a vessel feeding a lesion and visualization of direction of blood flow.

It will be apparent to those skilled in the art that additional parameters when adjusted dynamically during phi-motion, can be used in order to better visualize and identify feeder vessels and the direction of blood flow through them.

Upon receiving an instruction to start the display in phi-motion **340,** the angiographic images are displayed in phi-motion **350**. Display in phi-motion is effected by displaying the angiographic images of the defined subsequence in series and according to the dynamic parameters. In a preferred embodiment of the invention, the phi-motion is displayed in a display window on a monitor.

Changes to any of the dynamic parameters are detected **370** and updated **380** in real-time such that the phi-motion continues running. Therefore, by performing IPM, one can view the successive angiographic images displayed in phi-motion while dynamically changing parameters such as speed, interval, direction and pixel brightness in real-time.

Significantly, the present invention of IPM has several advantageous effects over and above conventional methods. First, by visualizing a dye filling up the vessels in a particular region in phi-motion, one can readily identify vessels in connection with the CNV and the direction of blood flow in them hence identifying the feeder (afferent) vessels as differentiated from the draining (efferent) vessels. Identification of FVs is essential to the success of photocoagulation as a treatment for lesions. Second, improved visualization and identification of FVs is accomplished by being able to manipulate the dynamic parameters in real-time while phi-motion display of the angiographic images continue to loop. Manipulation of these four parameters (speed, interval, direction and pixel brightness) dramatically improves the ability to visualize the dye entering the vessels in the region of interest and thus enhances FV identification. Finally, all the above is accomplished by obtaining one high-speed set of angiographic images. There is no need to take separate successive angiograms, possibly with multiple boluses, while trying to adjust the parameters between captures based on the previous angiogram set.

In another embodiment of the invention, sequential subtraction of images in the defined subsequence is effected prior to running the phi-motion in order to reduce noise. Sequential subtraction may be performed with registration which determines the amount of shift and rotation between successive images, allowing successive images to be aligned and thus optimizing sequential subtraction. In order to further reduce noise, a Fourier Filter can be effected on the subtracted images. Methods relating to sequential subtraction, registration Fourier filtering, as is disclosed by U.S. Patent No. 5,394,199 are well known in the art.

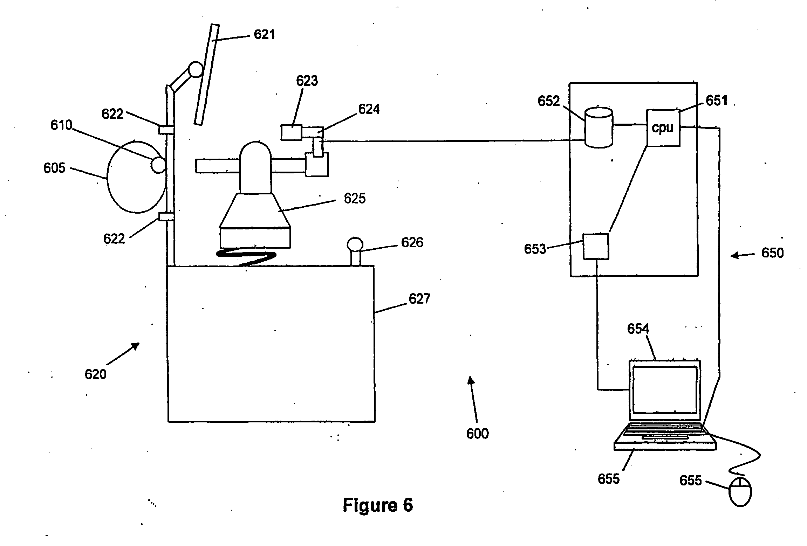

Referring to figure 6, there is shown an overall system overview diagram of a preferred embodiment of the invention incorporating the use of IPM 600. In this embodiment, a subject's head 605 containing the subject's eye 610 is shown. An apparatus operationally disposed to capture high-speed images 620 is comprised of a viewing monitor 621, a head mount 622, a CCD camera 623, a camera mount adaptor 624, a modified fundus camera 625, a camera positioning control 626 and a power and instrumentation cabinet 627. A computer system 650 comprises a CPU 651, memory 652, such as a hard disk and random access memory, an imaging processor 653, a PC monitor 654and one or more input devices 655.

In practice, the subject's eye 610 contains the lesion of interest, such as an AMD-related CNV. As such, the subject's eye is a candidate for treatment by photocoagulation of the FV of the CNV. The subject places his/her head 605 into the head mount 622 which can be adjusted to align the subject with the rest of the apparatus 620. The camera positioning control 626 is used to adjust the modified fundus camera 625 in order to align the fundus camera 625 with the area of interest containing the CNV. This alignment is visualized using the viewing monitor 621, which is mounted on the head mount 622 and displays the view through the fundus camera 625.

Once the subject has been administered a visualizing dye such as ICG, the CCD camera 623, mounted via a camera mount adaptor 624, takes a series of high-speed angiographic images which are stored in memory 652 on computer system 650. The conversion of analog images to digital form, both generally and through the use of imaging board buffers (i.e. EPIX< >), are well known in the art. Each series of high-speed images are given a unique identifier such that it may be retrieved from memory 652. Each image in a series is also given a unique identifier such that it may be retrieved from memory 652.

A series of high-speed images are retrieved from memory 652, pursuant to a request by a user through one or more input devices 655, and displayed on the PC monitor 654 using image processor 653. The computer system 650, contains an operating system capable of performing run-time operations. A computer program is stored in memory **652** which, when executed by the CPU **651,** is comprised of instructions corresponding to the method of IPM **200.** The user defines a subsequence to undergo IPM via input devices **655.** Dynamic parameters are defined using one or more input devices **655.** In response to a start signal by the user through an input device **655,** the CPU **651** displays the subsequence in phi-motion, pursuant to the dynamic parameters, on the PC monitor **654** using image processor **653.** Dynamic parameters are updated by the CPU **651** during phi-motion.

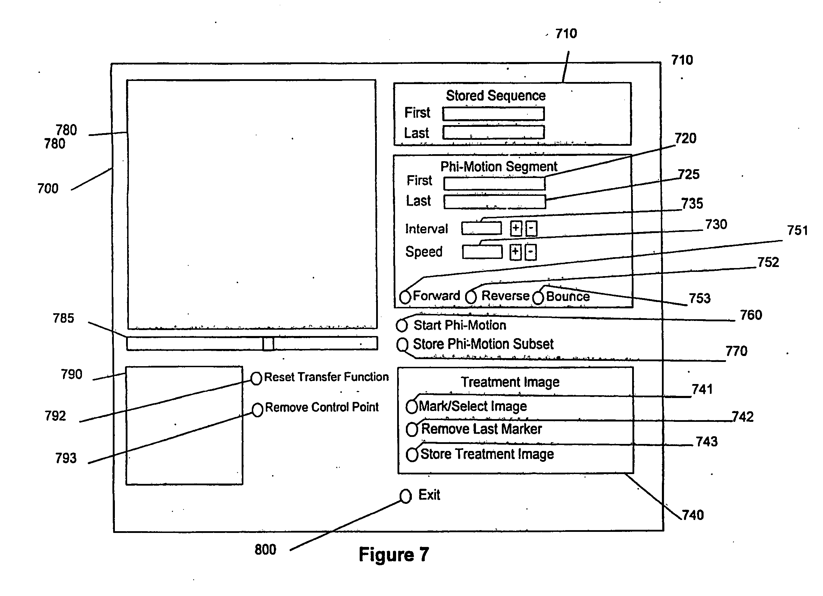

Referring to figure 7, a graphical user interface ("GUI") **700** of one embodiment of the invention is shown. The GUI **700** is displayed on the PC monitor **654.** The GUI **700** comprises a display window **780,** a corresponding slider bar 785, a LUT display **790,** a stored sequence window **710,** a phi-motion segment window **715,** a treatment image window **740,** a start phi-motion button and a store phi-motion subset button.

The display window **780** displays images in the stored sequence. The display window **780** also displays the subsequence in phi-motion when the phi-motion display is running. The slider bar **785** is used select individual angiographic images in the stored sequence or subsequence to be displayed. The LUT display **790** is the interface used by the user to manipulate the relationship between collected and displayed intensity values for the phi-motion subsequence referred to with respect to figures 3-5. The default relationship between collected and displayed intensity values, namely a directly proportional relationship, can be restored by selecting a reset transfer function button **792.** Control points may be added to the LUT by right clicking a mouse on an LUT line segment within the LUT display **790.** Control points may be removed by selecting a remove control point button **793.** Control points are automatically removed in the reverse order of their placement. Upon removal, addition or adjustment of each control point, the LUT is re-calculated with the modified points.

The stored sequence window **710** numerically displays the first and last frame numbers in the stored sequence. The phi-motion window **715** comprises the following areas in which user may input limits and parameters. The phi-motion subsequence is defined by a first and last image and by inputting the corresponding frame numbers into first **720** and last **725** image boxes in the phi-motion segment window **715**. The speed parameter is entered in a speed parameter box **730** in frames per second. The interval parameter is entered in an interval parameter box 735. The direction parameter is defined by selecting an appropriate direction button, forward 751, reverse 752, or bounce 753.

Phi-motion display can be initiated by selecting the start phi-motion button 760, and a phi-motion subsequence with accompanying parameters may be saved to memory 652 by selecting the store phi-motion subset button 770.

Treatment images may be manipulated by using the treatment image window 740. An individual treatment image may be marked with a marker by selecting a mark/select image button 741 and using the mouse to click on the treatment image displayed in the display window 780. The most recently added marker may be removed by selecting the remove last marker button 742. An individual treatment image may be stored in memory 652 by selecting a store treatment image button 743. Finally, a user may exit the program by selecting an exit button 800.

Although the invention has been described with reference to certain specific embodiments, various modifications thereof will be apparent to those skilled in the art as outlined in the claims appended hereto.

## Claims

1. A method for visualizing the direction of fluid flow through vessels having a visualizing composition comprising a fluorescent dye flowing therethrough said method comprising the steps of:(a) selecting from a sequence of angiographic images a subsequence of angiographic images; (b) reading a plurality of dynamic parameters, said dynamic parameters (330) for controlling display of a series of angiographic images (350); (c) serially displaying the subsequence of angiographic images repetitively in accordance with said dynamic parameters; and **characterized by** (d) providing an interface (650) allowing for dynamic user update (380) of said dynamic parameters while repeatedly displaying said subsequence in a continuous loop.

2. The method according to claim 1, wherein one of said dynamic parameters comprises a speed parameter (730), said speed parameter defining a speed at which said sequence is serially displayed.

3. The method according to any of claims 1-2, wherein one of said dynamic parameters comprises an interval parameter, said interval parameter defining a number "n" corresponding to serially displaying said sequence by serially displaying every n^{th} angiographic image.

4. The method according to any of claims 1-3, wherein one of said dynamic parameters comprises a direction parameter (751, 752), said direction parameter defining a direction in which said sequence is serially displayed.

5. The method according to any of claims 1-4, wherein one of said dynamic parameters comprises a pixel brightness function, said pixel brightness function representing a relationship between an original pixel intensity value and a displayed pixel intensity value, and wherein said pixel brightness function is represented by a Look-Up table capable of being manipulated by said user.

6. The method according to any of claims 1-5, wherein said visualizing composition comprises indocyanine green dye, or further comprising exciting the fluorescent dye with radiation at a wavelength between 780-830nm.

7. The method according to any of claims 1-6, wherein one of said vessels is a blood vessel feeding blood to a lesion., or wherein one of said vessels is a blood vessel feeding blood to an age-related macular degeneration associated choroidal neovascularization.

8. A method according to any of claims 1 to 7 wherein the continuous loop is a continuous forward loop, a continuous backward loop, or a continuous bounce.

9. An apparatus for visualizing the direction of fluid flow through vessels having a visualizing composition comprising a fluorescent dye flowing therethrough, comprising: (a) a means for selecting from a sequence of angiographic images a subsequence of angiographic images; (b) a means for reading a plurality of dynamic parameters, said dynamic parameters (330) for controlling display of a sequence of angiographic images (350); (c) a means for serially displaying said subsequence repetitively in accordance with said dynamic parameters; and **characterized by** (d) a means for providing an interface (650) allowing for dynamic user update (380) of said dynamic parameters while repeatedly displaying said subsequence in a continuous loop.

10. The apparatus according to claim 9, wherein one of said dynamic parameters comprises a speed parameter (730), said speed parameter defining a speed at which said sequence is serially displayed.

11. The apparatus according to any of claims 9-10, wherein one of said dynamic parameters comprises an interval parameter, said interval parameter defining a number "n" corresponding to serially displaying said sequence by serially displaying every n^{th} angiographic image.

12. The apparatus according to any of claims 9-11, wherein one of said dynamic parameters comprises a direction parameter (751, 752), said direction parameter defining a direction in which said sequence is serially displayed.

13. The apparatus according to any of claims 9-12, wherein one of said dynamic parameters comprises a pixel brightness function, said pixel brightness function representing a relationship between an original pixel intensity value and a displayed pixel intensity value, and preferably wherein said pixel brightness function is represented by a Look-Up table capable of being manipulated by said user.

14. The apparatus according to any of claims 9-13, for visualizing the direction of fluid flow through vessels having a visualizing composition comprising indocyanine green dye, further arranged to excite the fluorescent dye with radiation at a wavelength between 780-830nm.

15. A computer program product, comprising: a memory having computer-readable code embodied therein for implementing the method of any of claims 1 to 8.

## Patentansprüche

1. Verfahren zum Sichtbarmachen der Richtung eines Fluidstroms durch Gefäße mit einer durch diese hindurchfließenden Visualisierungs-Zusammensetzung, die einen fluoreszierenden Farbstoff aufweist, welches Verfahren die Schritte umfasst: (a) Auswählen einer Teilsequenz angiographischer Bilder aus einer Sequenz angiographischer Bilder; (b) Lesen einer Vielzahl dynamischer Parameter, welche dynamischen Parameter (330) zum Steuern der Anzeige einer Serie angiographischer Bilder (350) dienen; (c) wiederholtes serielles Anzeigen der Teilsequenz der angiographischen Bilder gemäß den dynamischen Parametern; und **gekennzeichnet durch** (d) Bereitstellen einer Schnittstelle (650), die eine dynamische Benutzeraktualisierung (380) der dynamischen Parameter ermöglicht, während die Teilsequenz in einer Endlosschleife wiederholt angezeigt wird.

2. Verfahren nach Anspruch 1, wobei einer der dynamischen Parameter einen Geschwindigkeits-Parameter (730) umfasst, welcher Geschwindigkeits-Parameter eine Geschwindigkeit festlegt, mit welcher die Sequenz seriell angezeigt wird.

3. Verfahren nach einem der Ansprüche 1-2, wobei einer der dynamischen Parameter einen Intervall-Parameter umfasst, welcher Intervall-Parameter eine Anzahl "n" festlegt, die der seriellen Anzeige der Sequenz entspricht, indem jedes n-te angiographische Bild seriell angezeigt wird.

4. Verfahren nach einem der Ansprüche 1-3, wobei einer der dynamischen Parameter einen Richtungs-Parameter (751, 752) umfasst, welcher Richtungs-Parameter eine Richtung festlegt, in welcher die Sequenz seriell angezeigt wird.

5. Verfahren nach einem der Ansprüche 1-4, wobei einer der dynamischen Parameter eine Pixelhelligkeitsfunktion umfasst, welche Pixelhelligkeitsfunktion eine Beziehung zwischen einem ursprünglichen Pixelintensitätswert und einem angezeigten Pixelintensitätswert repräsentiert, und wobei die Pixelhelligkeitsfunktion durch eine Verweistabelle repräsentiert ist, die durch den Benutzer manipuliert werden kann.

6. Verfahren nach einem der Ansprüche 1-5, wobei die Visualisierungs-Zusammensetzung Indocyaningrün-Farbstoff umfasst, oder welches weiters das Anregen des fluoreszierenden Farbstoffs mit einer Strahlung bei einer Wellenlänge zwischen 780-830nm umfasst.

7. Verfahren nach einem der Ansprüche 1-6, wobei eines der Gefäße ein Blutgefäß ist, das einer Läsion Blut zuführt, oder wobei eines der Gefäße ein Blutgefäß ist, das einer mit einer altersbedingten Makuladegeneration verbundenen choroidalen Neovaskularisation Blut zuführt.

8. Verfahren nach einem der Ansprüche 1-7, wobei die Endlosschleife eine endlose Vorwärtsschleife, eine endlose Rückwärtsschleife oder ein endloses Hin- und Herspringen ("bounce") ist.

9. Vorrichtung zum Sichtbarmachen der Richtung eines Fluidstroms durch Gefäße hindurch mit einer durch diese hindurchfließenden Visualisierungs-Zusammensetzung, welche einen fluorezierenden Farbstoff umfasst, mit: (a) einem Mittel zum Auswählen einer Teilsequenz angiographischer Bilder aus einer Sequenz angiographischer Bilder; (b) einem Mittel zum Lesen einer Vielzahl dynamischer Parameter, welche dynamischen Parameter (330) zum Steuern der Anzeige einer Sequenz angiographischer Bilder (350) dienen; (c) einem Mittel zum wiederholten seriellen Anzeigen der Teilsequenz gemäß den dynamischen Parametern; und **gekennzeichnet durch** (d) ein Mittel zum Bereitstellen einer Schnittstelle (650), die eine dynamische Benutzeraktualisierung (380) der dynamischen Parameter ermöglicht, währen die Teilsequenz in einer Endlosschleife wiederholt angezeigt wird.

10. Vorrichtung nach Anspruch 9, wobei einer der dynamischen Parameter einen Geschwindigkeits-Parameter (730) umfasst, welcher Geschwindigkeits-Parameter eine Geschwindigkeit festlegt, mit welcher die Sequenz seriell angezeigt wird.

11. Vorrichtung nach einem der Ansprüche 9-10, wobei einer der dynamischen Parameter einen Intervall-Parameter umfasst, welcher Intervall-Parameter eine Anzahl "n" festlegt, die der seriellen Anzeige der Sequenz entspricht, indem jedes n-te angiographische Bild seriell angezeigt wird.

12. Vorrichtung nach einem der Ansprüche 9-11, wobei einer der dynamischen Parameter einen Richtungs-Parameter (751, 752) umfasst, welcher Richtungs-Parameter eine Richtung festlegt, in welcher die Sequenz seriell angezeigt wird.

13. Vorrichtung nach einem der Ansprüche 9-12, wobei einer der dynamischen Parameter eine Pixelhelligkeitsfunktion umfasst, welche Pixelhelligkeitsfunktion eine Beziehung zwischen einem ursprünglichen Pixelintensitätswert und einem angezeigten Pixelintensitätswert repräsentiert, und wobei vorzugsweise die Pixelhelligkeitsfunktion durch eine Verweistabelle repräsentiert ist, die durch den Benutzer manipuliert werden kann.

14. Vorrichtung nach einem der Ansprüche 9-13 zum Sichtbarmachen der Richtung eines Fluidstroms durch Gefäße hindurch mit einer Visualisierungs-Zusammensetzung, welche Indocyaningrün-Farbstoff umfasst, welche weiters eingerichtet ist, den fluoreszierenden Farbstoff mit einer Strahlung bei einer Wellenlänge zwischen 780-830nm anzuregen.

15. Computerprogrammprodukt umfassend: einen Speicher mit einem darin enthaltenen computerlesbaren Code zur Durchführung des Verfahrens nach einem der Ansprüche 1 bis 8.

## Revendications

1. Procédé destiné à visualiser la direction d'écoulement d'un fluide à travers des vaisseaux ayant une composition de visualisation comprenant un colorant fluorescent s'écoulant à travers eux, ledit procédé comprenant les étapes consistant à : (a) choisir à partir d'une séquence d'images angiographiques une sous-séquence d'images angiographiques ; (b) lire une pluralité de paramètres dynamiques, lesdits paramètres dynamiques (330) servant à contrôler l'affichage d'une série d'images angiographiques (350) ; (c) afficher en série la sous-séquence d'images angiographiques répétitivement en fonction desdits paramètres dynamiques ; et **caractérisé en ce qu'**il consiste à (d) fournir une interface (650) permettant la mise à jour dynamique par l'utilisateur (380) desdits paramètres dynamiques tout en affichant de manière répétée ladite sous-séquence sous forme de boucle continue.

2. Procédé selon la revendication 1, dans lequel un desdits paramètres dynamiques comprend un paramètre de vitesse (730), ledit paramètre de vitesse définissant une vitesse à laquelle ladite séquence est affichée en série.

3. Procédé selon l'une quelconque des revendications 1 et 2, dans lequel un desdits paramètres dynamiques comprend un paramètre d'intervalle, ledit paramètre d'intervalle définissant un nombre « n » correspondant à l'affichage en série de ladite séquence par l'affichage en série de chaque n^{ième} image angiographique.

4. Procédé selon l'une quelconque des revendications 1 à 3, dans lequel un desdits paramètres dynamiques comprend un paramètre de direction (751, 752), ledit paramètre de direction définissant une direction dans laquelle ladite séquence est affichée en série.

5. Procédé selon l'une quelconque des revendications 1 à 4, dans lequel un desdits paramètres dynamiques comprend une fonction de luminosité de pixel, ladite fonction de luminosité de pixel représentant une relation entre une valeur d'intensité de pixel d'origine et une valeur d'intensité de pixel affiché, et dans lequel ladite fonction de luminosité de pixel est représentée par une table de conversion pouvant être manipulée par ledit utilisateur.

6. Procédé selon l'une quelconque des revendications 1 à 5, dans lequel ladite composition de visualisation comprend un colorant de vert d'indocyanine, ou consistant en outre à exciter le colorant fluorescent par rayonnement à une longueur d'onde entre 780 et 830 nm.

7. Procédé selon l'une quelconque des revendications 1 à 6, dans lequel l'un desdits vaisseaux est un vaisseau sanguin alimentant en sang une lésion, ou dans lequel l'un desdits vaisseaux est un vaisseau sanguin alimentant en sang une dégénérescence maculaire liée à l'âge associée à la néovascularisation choroïdienne.

8. Procédé selon l'une quelconque des revendications 1 à 7, dans lequel la boucle continue est une boucle continue vers l'avant, une boucle continue vers l'arrière ou un rebond continu.

9. Appareil destiné à visualiser la direction d'écoulement d'un fluide à travers des vaisseaux ayant une composition de visualisation comprenant un colorant fluorescent s'écoulant à travers eux, comprenant : (a) des moyens destinés à choisir à partir d'une séquence d'images angiographiques une sous-séquence d'images angiographiques ; (b) des moyens destinés à lire une pluralité de paramètres dynamiques, lesdits paramètres dynamiques (330) servant à contrôler l'affichage d'une séquence d'images angiographiques (350) ; (c) des moyens destinés à afficher en série ladite sous-séquence répétitivement en fonction desdits paramètres dynamiques ; et **caractérisé par** (d) des moyens destinés à fournir une interface (650) permettant la mise à jour dynamique par l'utilisateur (380) desdits paramètres dynamiques tout en affichant de manière répétée ladite sous-séquence dans une boucle continue.

10. Appareil selon la revendication 9, dans lequel un desdits paramètres dynamiques comprend un paramètre de vitesse (730), ledit paramètre de vitesse définissant une vitesse à laquelle ladite séquence est affichée en série.

11. Appareil selon l'une quelconque des revendications 9 et 10, dans lequel l'un desdits paramètres dynamiques comprend un paramètre d'intervalle, ledit paramètre d'intervalle définissant un nombre « n » correspondant à l'affichage en série de ladite séquence en affichant en série chaque n^{ième} image angiographique.

12. Appareil selon l'une quelconque des revendications 9 à 11, dans lequel l'un desdits paramètres dynamiques comprend un paramètre de direction (751, 752), ledit paramètre de direction définissant une direction dans laquelle ladite séquence est affichée en série.

13. Appareil selon l'une quelconque des revendications 9 à 12, dans lequel l'un desdits paramètres dynamiques comprend une fonction de luminosité de pixel, ladite fonction de luminosité de pixel représentant une relation entre une valeur d'intensité de pixel d'origine et une valeur d'intensité de pixel affiché, et dans lequel de préférence, ladite fonction de luminosité de pixel est représentée par une table de conversion capable d'être manipulée par ledit utilisateur.

14. Appareil selon l'une des revendications 9 à 13, permettant de visualiser la direction d'écoulement d'un fluide à travers des vaisseaux ayant une composition de visualisation comprenant un colorant de vert d'indocyanine, conçu en outre pour exciter le colorant fluorescent par rayonnement à une longueur d'onde entre 780 et 830 nm.

15. Produit de programme informatique, comprenant : une mémoire dans laquelle est incorporée un code lisible par ordinateur pour mettre en oeuvre le procédé selon l'une des revendications 1 à 8.
